# EUROPÄISCHE PATENTSCHRIFT

(11) **EP 3 474 767 B1**
(45) Veröffentlichungstag und Bekanntmachung des Hinweises auf die Patenterteilung: **21.02.2024**
(21) Anmeldenummer: 17730772.5
(22) Anmeldetag: 14.06.2017
(51) Int. Cl.: A61B 34/30, A61B 34/00, A61B 90/50

(54) **INSTRUMENTENTRÄGERVORRICHTUNG FÜR EINEN MANIPULATOR EINES ROBOTISCHEN OPERATIONSSYSTEMS**
INSTRUMENT SUPPORT DEVICE FOR A MANIPULATOR OF A ROBOTIC SURGICAL SYSTEM
ENSEMBLE PORTE-INSTRUMENT POUR UN MANIPULATEUR DE SYSTÈME ROBOTISÉ POUR INTERVENTIONS CHIRURGICALES

(30) Priorität: 27.06.2016 DE 102016111737
(43) Veröffentlichungstag der Anmeldung: 01.05.2019
(73) Patentinhaber: avateramedical GmbH, 07745 Jena (DE)
(72) Erfinder: KARGUTH, Andreas, 99869 Tuettleben (DE); TROMMER, Christian, 99310 Wipfratal (DE)
(74) Vertreter: Gleim Petri Patent- und Rechtsanwaltspartnerschaft mbB
(86) Internationale Anmeldenummer: PCT/EP2017/064587
(87) Internationale Veröffentlichungsnummer: WO 2018/001742

(56) Entgegenhaltungen:
- WO-A1-2009/120945
- WO-A1-2014/094716
- DE-A1-102013 012 840
- US-A1- 2013 144 307
- US-A1- 2013 325 029

## Beschreibung

### Gebiet der Erfindung

Die Erfindung betrifft eine Instrumententrägervorrichtung für einen Manipulator eines robotischen Operationssystems. Diese Vorrichtung umfasst einen dreigliedrigen Arm als Endstück einer offenen kinematischen Kette mit einem ersten, zweiten und dritten Armglied. Ein Manipulator ist dabei derjenige Teil eines robotischen Systems, welches physikalisch mit seiner Umgebung wechselwirken kann, d.h. der bewegliche Teil des Systems. Dabei handelt es sich in der Regel um ein vielgliedriges System, bei dem die einzelnen Glieder - im Folgenden der Anschaulichkeit halber auch als Armglieder bezeichnet - über Gelenke miteinander verbunden sind. Ein Teil der Glieder dient der Positionierung des Manipulators relativ zu anderen Manipulatoren desselben robotischen Systems, so dass diese sich nicht gegenseitig behindern, und wird im Folgenden auch als Stellvorrichtung bezeichnet. Ein anderer Teil der Glieder - hier der Instrumententrägervorrichtung - dient der Positionierung des Werkzeugs am Objekt, also beispielsweise der Positionierung eines Operationsinstruments oder eines Endoskops im Bereich einer Öffnung der Gewebedecke eines Patienten, z.B. der Bauchdecke, für eine minimal-invasive Operation. Die Armglieder sind dabei als offene kinematische Kette miteinander verbunden, da am letzten Glied kein weiteres Gelenk sitzt, sondern das Werkzeug, und alle anderen Glieder nach Art einer Kette miteinander verbunden sind. An den Enden eines jeden Armgliedes innerhalb der Kette ist ein Gelenk, so dass jedes Armglied zwei Gelenke umfasst, und über die Gelenke sind die Armglieder mit anderen Armgliedern verbunden. Ein Gelenk verbindet dabei genau zwei Glieder. Die Gelenke erlauben den Gliedern eine eingeschränkte Bewegung entsprechend der dem Gelenk zur Verfügung stehenden Freiheitsgrade relativ zueinander, wobei an einem Glied mehrere Gelenke ausgebildet sein können. Außerdem können zwei Glieder auch durch mehr als ein Gelenk verbunden sein. Die Gelenke können entweder aktiv, d.h. motorisch angetrieben, oder passiv, d.h. freibeweglich, sein.

Der eingangs erwähnte dreigliedrige Arm bildet beim Manipulator den für die Positionierung des Instruments zuständigen Teil. Die Instrumententrägervorrichtung umfasst daher zunächst eine Schnittstelle zur Verbindung des ersten Armglieds mit dem Manipulator, d.h. der Stellvorrichtung des Manipulators. Diese Verbindung wird über ein Schnittstellenrotationsgelenk hergestellt. Unter einem Rotationsgelenk wird dabei ein Gelenk mit einer Rotationsachse verstanden, bei dem die Rotationsachse einen rechten Winkel mit den Achsen der beiden angeschlossenen Armglieder bildet. Die Achse eines Gliedes liegt dabei in dessen Längsrichtung, d.h. entlang der Richtung, in der das Glied zwei Gelenke verbindet bzw. beim End-Armglied zwischen offenem Ende und Gelenk. Im Gegensatz zum Rotationsgelenk verläuft bei einem Torsionsgelenk die Drehachse parallel zu den Achsen der beiden Armglieder, und bei einem Revolvergelenk verläuft die Längsachse des einen Gliedes parallel zur Drehachse und die Längsachse des anderen Gliedes - in der Regel des Ausgangsgliedes - steht im rechten Winkel zur Drehachse. Bei dem Schnittstellenrotationsgelenk handelt es sich um ein Rotationsgelenk, bei dem also die Drehachse mit den Achsen der beiden angeschlossenen Glieder einen rechten Winkel einschließt. Das erste Armglied ist mit dem zweiten Armglied über ein Torsionsgelenk verbunden. Das zweite und das dritte Armglied sind miteinander über ein erstes Rotationsgelenk verbunden, und das dritte Armglied ist mit einem Instrumentenhalter, welcher die Funktion eines vierten Armgliedes hat und das eigentliche Ende der kinematischen Kette bildet, mittels eines Schubgelenks verbunden, d.h. drittes Armglied und Instrumentenhalter sind entlang einer Schubachse, die hier mit der Längsachse des dritten Armglieds zusammenfällt, gegeneinander bewegbar. Der Instrumentenhalter dient der Aufnahme eines chirurgischen Instruments mit einer Instrumentenlängsachse. Die Rotationsachse des ersten Rotationsgelenks liegt senkrecht zur Rotationsachse des Torsionsgelenks.

Der Begriff der Instrumentenlängsachse ist dabei wie folgt zu verstehen: Bei der minimal-invasiven Chirurgie mittels eines robotischen Systems werden die für die Operation benötigten Instrumente, beispielsweise Zangen, Scheren oder Nadelhalter, über einen Stangenmechanismus mit Instrumentenstangen geführt. Zur Beobachtung des Operationsbereiches im Inneren des Körpers werden stabförmige Kamerasysteme, Endoskope mit Endoskopstangen eingesetzt. Die Instrumentenlängsachse entspricht daher der Längsachse solcher meist zylindrischen Instrumenten- bzw. Endoskopstangen.

Schließlich umfasst die Instrumententrägervorrichtung auch eine Steuerung mit Antrieben für das Torsionsgelenk, das erste Rotationsgelenk und das Schubgelenk zur Bewegung der Armglieder und des Instrumentenhalters relativ zueinander. Es handelt sich also bei diesen Gelenken um aktive Gelenke. Welches auch als Lineargelenk, Translationsgelenk oder prismatisches Gelenk bezeichnet wird, versteht man ein Gelenk, welches eine gleitende oder fortschreitende Bewegung entlang der Längsachse des einen Armglieds bewirkt.

Bei der minimal-invasiven Chirurgie mittels robotischer Operationssysteme wird die herkömmlich manuelle Führung der für die minimal-invasive Chirurgie geeigneten Instrumente durch eine motorische Positionierung ersetzt, wobei die Positionierung und die Operation von Hand durch einen Operateur durchgeführt werden. Bei Operationen im Innern des Körpers werden die Instrumentenstangen - im Folgenden zusammenfassend auch als Instrument bezeichnet - über einen oder mehrere Trokare in das Innere des Patienten geführt. Unter einem Trokar versteht man dabei ein Instrument, mit dessen Hilfe der Chirurg in der minimal-invasiven Operationstechnik einen Zugang zu einer Körperhöhle wie beispielsweise dem Bauchraum legt, der Trokar hat üblicherweise Schneidkanten für die scharfe Präparation eines Zugangs oder eine stumpfe konische Spitze zur stumpfen Präparation. Die Schneidkanten bzw. die Spitze bilden das vordere Ende eines Stiftes, der in einem Tubus sitzt, die Spitze des Trokars verschließt die Öffnung des Tubus. Der Tubus wird mit dem Trokar gemeinsam in die entsprechende Körperhöhle eingeführt, sodann wird der Trokar aus dem Tubus herausgezogen und es verbleibt nur der Tubus im Körper, durch welchen die Instrumentenstangen geführt werden. Unter dem Begriff des Trokars soll im Folgenden insbesondere auch dessen Tubus verstanden werden, welcher für die Positionierung des chirurgischen Instruments von wesentlicher Bedeutung ist, sofern er verwendet wird.

Grundsätzlich kann das chirurgische Instrument mit Hilfe des Manipulators beliebig in allen drei Raumrichtungen bewegt und positioniert werden, üblicherweise werden die Instrumente - mit oder ohne Tubus - jedoch so geführt, dass in einem Punkt der Gewebedecke keine oder nur minimale seitliche Bewegungen der Instrumentenstangen ausgeführt werden können. Dieser Punkt wird als Pivotpunkt, Angelpunkt oder Drehpunkt bezeichnet. Die Steuerungslogik des robotischen Operationssystems muss den Pivotpunkt kennen, bzw. der Pivotpunkt muss durch die konstruktive Ausführung der Bewegungsmechanik definiert sein, um die Bewegung des Instruments so zu begrenzen, dass die biomechanische Belastung des Gewebes um den Tubus bzw. den Durchstoßpunkt durch das Gewebe herum möglichst gering ist. Am Pivotpunkt, welcher idealerweise im Durchstoßpunkt des Trokars durch die Gewebedecke eines Patienten oder in der Nähe dieses Durchstoßpunktes liegt, müssen seitliche Bewegungen parallel zur Oberfläche der Gewebedecke konstruktions- und / oder steuerungsbedingt ausgeschlossen werden, um eine Verletzung der Gewebedecke zu verhindern. Die Instrumentenstangen dürfen in diesem Punkt daher nur um die Normale der Gewebedecke geschwenkt werden und in Richtung der Normalen verschoben werden, zusätzlich zu einer Rotation um die Instrumentenlängsachse zur Orientierung des Instruments.

### Stand der Technik

Im Stand der Technik sind verschiedene Instrumententrägervorrichtungen bzw. entsprechende Manipulatoren mit Instrumententrägen für robotische Operationssystem bekannt. In der EP 2 332 484 A2 wird eine Manipulationseinheit für die minimal-invasive Chirurgie beschrieben, welche einen mehrgliedrigen Arm umfasst, an dessen Ende ein Instrumentenhalter sitzt, der mittels eines Schubgelenks - auch als Lineargelenk bezeichnet - entlang einer Instrumentenlängsachse in vertikaler Richtung verschiebbar ist. Dieser ist über ein Rotationsgelenk mit einem weiteren Armglied verbunden, welches seinerseits über ein Torsionsgelenk mit einem anderen Armglied verbunden ist. Die Achse des Instrumentenhalters fällt hier mit der Instrumentenlängsachse zusammen bzw. läuft parallel zur dieser, das Lineargelenk verschiebt den Instrumentenhalter entlang dessen Längsachse. Um im Pivotpunkt seitliche Kräfte senkrecht zur Instrumentenlängsachse bzw. in der Gewebedecke zu unterbinden, sind für eine Verstellung des Instruments für eine Schwenkbewegung komplexe Ausgleichsbewegungen des gesamten Arms notwendig, um das Instrument in der Gewebedecke an der vorbestimmten Position zu halten. Bei der Verwendung von mehreren Manipulatoren steht unter Umständen nicht ausreichen Bewegungsfreiheit zur Verfügung, so dass es zu Kollisionen zwischen den Manipulatoren kommen kann. Durch die aktive Kopplung zwischen Instrumentenhalterung bzw. Trokar und Manipulator ist die Lage des Pivotpunkts immer zwingend gegeben.

Diese Nachteile werden durch eine in der WO 2014/094716 beschriebene Vorrichtung teilweise beseitigt. Hier ist eine Entkopplung der Instrumentenlängsachse von der Achse, entlang der eine teleskopische Verstellung erfolgt, realisiert, wodurch der für die Bewegung zur Positionierung des Instruments notwendige Raum verkleinert und die Kollisionsgefahr verringert wird. Zur Einstellung wird zum einen eine Teleskopführung mit Seilzugmechanismus verwendet, und zum anderen ein Koppelmechanismus mit sechs Lagerstellen, d.h. ein Doppelkoppel-Mechanismus. Insbesondere ist der Doppelkoppel-Mechanismus kompliziert aufgebaut und daher relativ störanfällig. Zusammen mit der Teleskopführung ist der benötigte Platz darüber hinaus recht groß.

US 2013/144307 A1 beschreibt eine Instrumententrägervorrichtung für einen Manipulator eines robotischen Operationssystems mit einem dreigliedrigen Arm.

### Beschreibung der Erfindung

Die Erfindung wird durch den beiliegenden unabhängigen Anspruch definiert. Bevorzugte Ausführungsformen der Erfindung sind in den abhängigen Ansprüchen offenbart.

Aufgabe der Erfindung ist es daher, eine Instrumententrägervorrichtung der eingangs beschriebenen Art dahingehend weiterzuentwickeln, dass diese zum einen möglichst robust, d.h. wenig störanfällig aufgebaut ist, zum anderen möglichst klein dimensioniert, um die Beweglichkeit zu erhöhen und außerdem im Verbund mit anderen Manipulatoren eine Kollisionsgefahr bei Bewegung der Manipulatoren zur Einstellung einer Operationsposition etc. die Kollisionsgefahr minimiert wird, so dass man größere Freiheiten für die Einstellung erhält.

Diese Aufgabe wird bei einer Instrumententrägervorrichtung der eingangs beschriebenen Art dadurch gelöst, dass der Instrumentenhalter mit dem dritten Armglied nicht nur über das Schubgelenk, sondern auch über ein zweites, passives Rotationsgelenk verbunden ist, um dessen Achse der Instrumentenhalter frei rotierbar ist, d.h. die Bewegung um dieses Gelenk wird nicht durch einen Antrieb gesteuert. Die Rotationsachsen des ersten und des zweiten Rotationsgelenks liegen zueinander parallel. Das zweite Rotationsgelenk befindet sind dabei vorzugsweise zwischen dem Schubgelenk und dem Instrumentenhalter, kann also beispielsweise an einem entsprechenden Schlitten, an den der Instrumentenhalter angekoppelt wird und der den Instrumentenhalter schiebt, angebracht sein. Grundsätzlich ist es auch möglich, jedoch konstruktionstechnisch aufwendiger, am dritten Armglied zunächst das zweite, passive Rotationsgelenk vorzusehen und an dieses das Schubgelenk mit dem Instrumentenhalter zu koppeln, im Endeffekt wird die gleiche Kinematik erreicht. Wesentlich ist, dass das dritte Armglied mit dem Instrumentenhalter nicht nur über das Schubgelenk, sondern auch über das zweite, passive Rotationsgelenk gekoppelt ist, d.h. also über zwei Gelenke. Auf diese Weise ist die Instrumentenlängsachse zwangsfrei durch einen Pivotpunkt führbar, welcher auf der Drehachse des Torsionsgelenks in einer Verlängerung aus dem zweiten Armglied heraus liegt. Im Unterschied zum Stand der Technik liegt der Pivotpunkt also nicht auf der Längsachse oder einer Verlängerung der Längsachse des dritten Armgliedes, sondern versetzt auf der Drehachse des Torsionsgelenks, jedoch außerhalb des zweiten Armgliedes und des ersten Rotationsgelenks, d.h. auf einer Verlängerung der Drehachse des Torsionsgelenks. Während bei einer Verstellung der Instrumententrägervorrichtung ein Längenversatz über die Achse des Schubgelenks, d.h. die Achse der Verschiebung mit kompensiert wird, so dient das zweite Rotationsgelenk dazu, eine Führung des Instruments - d.h. der Instrumentenstange - im Pivotpunkt ohne Zwang zu ermöglichen, d.h. ohne dass das Instrument seine Lage in der Ebene, die durch das Gewebe um die Körperöffnung herum definiert wird, verändern möchte und nur Schwenkbewegungen um den Pivotpunkt ausführt. Auf die Gewebedecke, beispielsweise die Bauchdecke eines Patienten, werden im Bereich der Öffnung keine seitlichen Kräfte in dieser Ebene ausgeübt. Während des Betriebs wird das Instrument, d.h. die Instrumentenlängsachse, durch diesen Pivotpunkt geführt. Abgesehen von der Schwenkbewegung sind selbstverständlich auch Bewegungen des Instruments entlang der Instrumentenlängsachse möglich sowie Drehungen um die Instrumentenlängsachse.

Während bei den im Stand der Technik bekannten Instrumententrägervorrichtungen das Instrument durch die spezielle Kinematik des Doppelkoppel-Antriebs stets um den Pivotpunkt zwangsgeführt wird, was bei nicht richtiger Systemeinrichtung, bei Patienten mit sehr dicken Gewebeschichten beispielsweise am Bauch, oder bei zwischenzeitlichen Verlagerungen des Patienten zu erheblichen Gewebebelastungen führen kann, werden bei der erfindungsgemäßen Instrumententrägervorrichtung Positionsveränderungen des Pivotpunktes, d.h. des Punktes, in welchem das Instrument das Gewebe durchtritt, passiv und / oder elastisch ausgeglichen.

Grundsätzlich ist für die Führung des Instruments, d.h. der Instrumentenstange, keine besondere Vorrichtung notwendig, die Körperöffnung, ggf. mit eingesetztem Tubus eines Trokars, ist als Lagerpunkt und Führung ausreichend, wenn es die Lage der Gewebedecke und der Öffnung darin zulässt, diese für die Definition des Pivotpunkts zu verwenden. In Fällen, wo es nicht möglich ist, einen solchen Angelpunkt mittels einer Gewebedecke des Körpers des Patienten zu erzeugen, ist es vorteilhaft den Pivotpunkt mechanisch auf andere Weise zu realisieren. Zu diesem Zweck ist am zweiten Armglied im Bereich des ersten Rotationsgelenks entlang der Verlängerung der Drehachse des Torsionsgelenks eine Trokarhalterung angeordnet, welche zur schwenkbaren Lagerung eines Trokars um eine zur Rotationsachse des ersten Rotationsgelenks parallele Schwenkachse ausgebildet ist. Die Halterung kann beispielsweise gabelförmig sein und der Tubus des Trokars kann in diese Halterung eingeklemmt oder mit ihr schwenkbar verschraubt werden, um nur zwei beispielhafte Möglichkeiten zur Herstellung der Lagerung zu nennen. Der Pivotpunkt ist dann der Schnittpunkt der Drehachse des Torsionsgelenks mit der Schwenkachse. Die Verwendung einer Trokarhalterung ist insbesondere dann vorteilhaft, wenn beispielsweise freie Operationen durchgeführt werden sollen, bei denen keine Gewebedecke, mit der ein Pivotpunkt definiert werden könnte, zur Verfügung steht. Die Trokarhalterung kann darüber hinaus auch als Zielhilfe verwendet werden um beispielsweise die Instrumententrägervorrichtung für eine Grundpositionierung in Bezug auf den Patienten einzurichten, ohne dass ein Trokar während der Operation verwendet wird.

Anstelle der im Stand der Technik bekannten Doppelkoppel wird hier ein Schubgelenk in Kombination mit einem freien Rotationsgelenk verwendet, das Schubgelenk kann beispielsweise als Linearführung mit Riemen- und / oder Spindelgetriebe ausgestaltet sein, was eine kompakte Bauform und eine gegen äußere mechanische Einflüsse robuste Konstruktion ermöglicht. Die Position des Schubgelenks entlang einer Schubgelenkachse, d.h. der Achse, entlang welcher die Verschiebung stattfindet, ist vorteilhaft mittels Relativ-Encoder und entlang der Schubgelenkachse angeordneten Referenzlagengebern bestimmbar. Auf diese Weise kann die Bauform besonders kompakt gehalten werden und indem im Bereich, in welchem die Verschiebung stattfindet, verschiedene Referenzschalter angeordnet sind, lässt sich die Referenzierung nach dem Einschalten auf ein Minimum reduzieren.

Der Instrumentenhalter schließlich kann auf verschiedene Weise an das dritte Armglied bzw. dessen Schubvorrichtung angekoppelt werden. Es kann sich beispielsweise um einen rein mechanischen Rastmechanismus handeln, was ein schnelles Auswechseln des Instrumentenhalters ermöglicht, wenn beispielsweise zwei Instrumente gewechselt werden müssen. Elektrische bzw. elektromagnetische Kontakte zur Steuerung des Instruments können in diesen Rastmechanismus integriert werden, die Steuersignale können aber auch per Funksignal übertragen werden.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Kurze Beschreibung der Zeichnungen

Nachfolgend wird die Erfindung beispielsweise anhand der beigefügten Zeichnungen, die auch erfindungswesentliche Merkmale offenbaren, noch näher erläutert. Es zeigen:
- Fig. 1: einen Manipulator eines robotischen Operationssystems in der Gesamtansicht,
- Fig. 2: die Kinematik dieses Manipulators,
- Fig. 3a,: b eine Instrumententrägervorrichtung in zwei verschiedenen Stellungen von der Seite,
- Fig. 4a,: b eine Instrumententrägervorrichtung von einer anderen Seite in zwei verschiedenen Stellungen,
- Fig. 5: eine Instrumententrägervorrichtung mit angekoppeltem Instrumentenhalter,
- Fig. 6a, b, c: eine weitere Ausgestaltung der Instrumententrägervorrichtung mit Instrumentenhalter, und
- Fig. 7a,: b eine Instrumententrägervorrichtung mit einem anderen Instrumentenhalter.

### Ausführliche Beschreibung der Zeichnungen

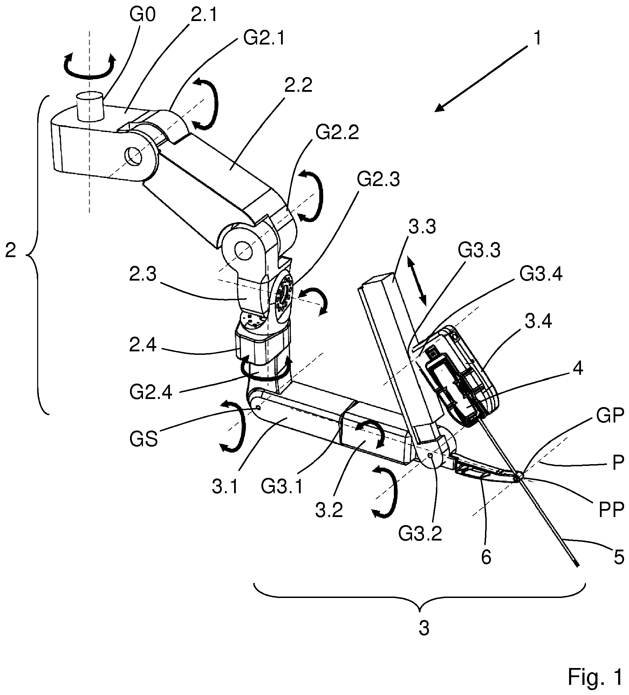

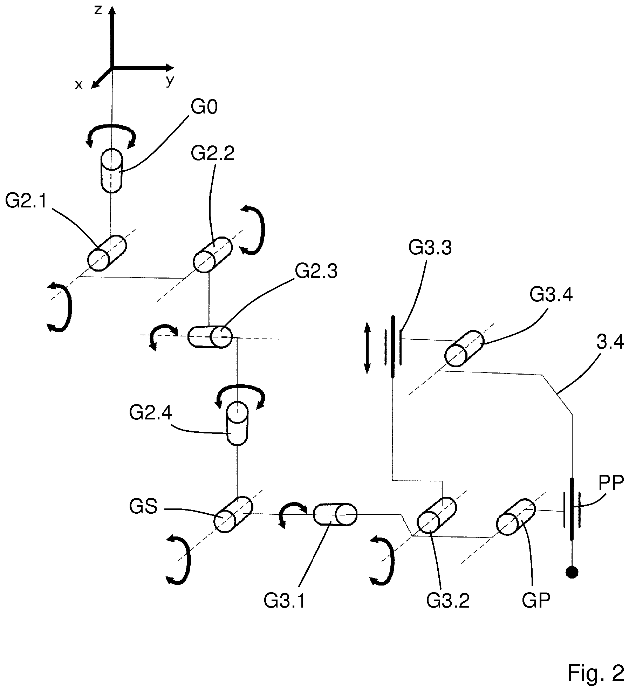

Fig. 1 zeigt einen Manipulator 1 eines robotischen Operationssystems und Fig. 2 die kinematischen Verhältnisse bei dem Manipulator 1. Ein robotisches Operationssystem umfasst in der Regel mehrere Manipulatoren, beispielsweise vier oder sechs. Der Manipulator 1 besteht aus einer Stellvorrichtung 2 und einer Instrumententrägervorrichtung 3. Die Stellvorrichtung 2 dient dazu, im Verbund mit den anderen Manipulatoren die Instrumententrägervorrichtung 3 so zu positionieren, dass die Instrumententrägervorrichtungen der verschiedenen Manipulatoren sich bei der Operation nicht gegenseitig behindern. Die Position des Instruments wird an der Instrumententrägervorrichtung 3 während der Operation und für diese eingestellt, die Stellvorrichtung 2 bleibt während der Operation üblicherweise in ihrer Position. Die Stellvorrichtung 2 besteht aus mehreren Stellarmgliedern 2.1, 2.2, 2.3 und 2.4. Das Stellarmglied 2.1 ist über ein Drehgelenk G0 mit dem übrigen, üblicherweise nicht beweglichen Teil des robotischen Operationssystems verbunden, wobei das Gesamtsystem selbst ggf. verfahrbar sein kann. Über angetriebene und angesteuerte Gelenke G2.1, G2.2, G2.3 und G2.4 lassen sich die Glieder der Stellvorrichtung 2, die Stellarmglieder 2.1 bis 2.4, gegeneinander bewegen.

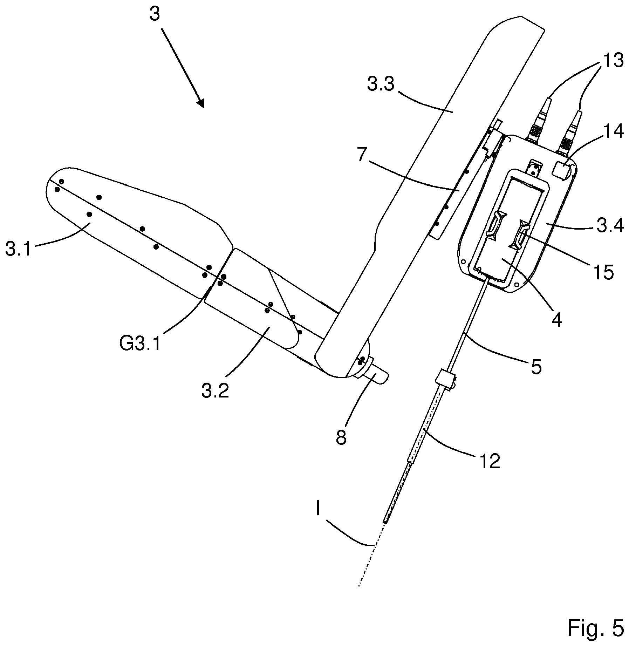

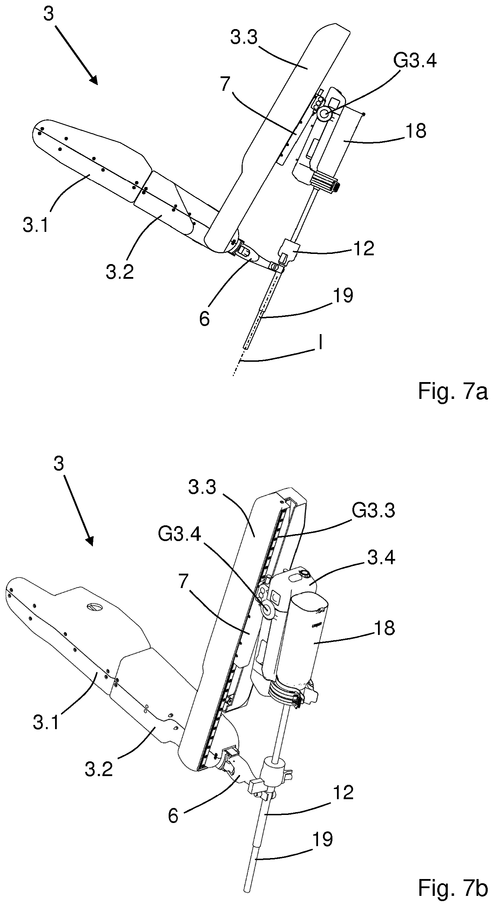

Die Instrumententrägervorrichtung 3 ihrerseits umfasst einen dreigliedrigen Arm als Endstück einer offenen kinematischen Kette mit einem ersten Armglied 3.1, einem zweiten Armglied 3.2 und einem dritten Armglied 3.3. Über eine Schnittstelle ist das erste, proximale Armglied 3.1 mit dem Manipulator 1, d.h. der Stellvorrichtung 2 des Manipulators 1 verbunden. Durch die Schnittstelle werden auch die elektrischen Kontakte für die Steuerung gelegt, sofern die Steuerung nicht per Funk erfolgt. Die Schnittstelle umfasst außerdem ein Schnittstellenrotationsgelenk GS, dessen Drehachse somit senkrecht zu den Längsachsen des ersten Armglieds 3.1 und des dem erste Armglied am nächsten angeordnete Stellarmglieds 2.4 ist. Das erste Armglied 3.1 der Instrumententrägervorrichtung 3 ist mit dem zweiten Armglied 3.2 über ein Torsionsgelenk G3.1 verbunden. Der Bewegungsbereich des Torsionsgelenks G3.1 liegt beispielsweise in einem Bereich von ±120°, bevorzugt von ±100° um eine Nulllage bzw. Ruhestellung. Das zweite Armglied 3.2 ist mit dem dritten, distalen Armglied 3.3 über ein erstes Rotationsgelenk G3.2 verbunden, dessen Bewegungsbereich beispielsweise zwischen -40° und +90°, bevorzugt zwischen -30° und +70° um eine Nulllage bzw. Ruhestellung liegt. In der Ruhestellung des Torsionsgelenks G3.1 liegen die Drehachsen des Schnittstellenrotationsgelenks GS und des ersten Rotationsgelenks G3.2 parallel. In der Ruhestellung des ersten Rotationsgelenks steht das dritte Armglied 3.3 bzw. dessen Längsachse senkrecht zur Drehachse des Torsionsgelenks G3.1 und zur Drehachse des ersten Rotationsgelenks G3.2. Das dritte Armglied 3.3 seinerseits ist über ein Schubgelenk G3.3 mit einem Instrumentenhalter 3.4 verbunden. Der Instrumentenhalter 3.4 sollte mindestens um einen Längenbereich von 30 cm, bevorzugt mindestens 44 cm, verstellbar sein, wobei dieser Längenbereich mit der Gesamtgröße des Operationssystems skaliert; die obige Angabe bezieht sich auf Systeme für minimalinvasive Eingriffe am menschlichen Körper. Der Instrumentenhalter 3.4 dient der Aufnahme eines chirurgischen Instruments, welches einen Instrumentenkopf 4 und eine Instrumentenstange 5 umfasst. Unter einem chirurgischen Instrument soll auch ein Endoskop verstanden werden, welches in der Chirurgie beobachtend eingesetzt wird. Im Instrumentenkopf 4 ist die Steuerung und ein Teil der Mechanik für die Bewegung der Instrumententeile untergebracht, die Instrumentenstange 5 wird in der Regel durch eine künstlich erzeugte Körperöffnung in der Gewebedecke des Patienten, beispielsweise durch die Bauchdecke, in den Körper eingeführt. Die Instrumentenstange 5 definiert gleichzeitig eine Instrumentenlängsachse I - gezeigt beispielsweise in den nicht perspektivischen Zeichnungen Fig. 5 und Fig. 7a -, d.h. die Instrumentenlängsachse I entspricht der Symmetrieachse der Instrumentenstange 5, bei beispielsweise zylinderförmigen Stangen. Unter einem chirurgischen Instrument werden dabei nicht nur Scheren, Nadeln, etc. verstanden, sondern auch Geräte zur Beobachtung des zu operierenden Bereichs wie beispielsweise Endoskope, ein entsprechender Instrumentenhalter 3.4 für ein solches Endoskop 18 mit einer Endoskopstange 19 ist beispielsweise in Fig. 7a, b gezeigt.

Die Instrumententrägervorrichtung 3 verfügt außerdem über eine nicht gezeigte Steuerung mit Antrieben für das Torsionsgelenk G3.1, das erste Rotationsgelenk G3.2 und das Schubgelenk G3.3 zur Bewegung der drei Armglieder 3.1, 3.2 und 3.3 und des Instrumentenhalters 3.4 relativ zueinander.

Das besondere bei der gezeigten Instrumententrägervorrichtung 3 ist, dass der Instrumentenhalter 3.4 mit dem dritten Armglied 3.3 nicht ausschließlich über das angetriebene Schubgelenk G3.3, sondern zusätzlich noch über ein zweites Rotationsgelenk G3.4 verbunden ist. Bei diesem zweiten Rotationsgelenk G3.4 handelt es sich um ein passives Gelenk, es wird also nicht angetrieben oder durch eine Steuerung bewegt, sondern ist um die Rotationsachse des Gelenks innerhalb gewisser, baubedingter Grenzen im Prinzip frei rotierbar. Der Bewegungsbereich bzw. Schwenkwinkel des zweiten Rotationsgelenks G3.4 beträgt beispielsweise etwa ±9° um eine mittlere Lage. Die Rotationsachsen sämtlicher Gelenke sind in Fig. 1 und Fig. 2 durch gestrichelte Linien gekennzeichnet, die Dreh- und Schubbewegungsmöglichkeiten bei den angetriebenen Gelenken durch entsprechende Doppelpfeile. Der Instrumentenhalter 3.4 ist also mit dem dritten Armglied 3.3 über zwei Gelenke verbunden, zum einen über das Schubgelenk G3.3 und zum anderen über das zweite Rotationsgelenk G3.4.

Die Rotationsachsen des ersten Rotationsgelenks G3.2 und des zweiten Rotationsgelenks G3.4 liegen zueinander parallel. Auf diese Weise ist die Instrumentenlängsachse I, dargestellt auch durch die Instrumentenstange 5, zwangsfrei durch einen Pivotpunkt PP führbar, welcher nicht im ersten Rotationsgelenk G3.2 oder auf der Längsachse des dritten Armglieds 3.3 liegt, sondern auf der Drehachse des Torsionsgelenks G3.1 in einer Verlängerung aus dem zweiten Armglied 3.2 heraus. Die Lage der Instrumentenlängsachse I bzw. der Instrumentenstange 5 ist also von der Lage der Längsachse des Armglieds 3.3 teilweise entkoppelt und die Rotationsachse des ersten Rotationsgelenks G3.2 verläuft nicht durch den Pivotpunkt PP. Ein Längenversatz wird über die Schubachse, d.h. die Längsachse des Armglieds 3.3, kompensiert und eine zwangsfreie Führung des Instruments wird durch die freie Drehbewegung zwischen dem Instrumentenhalter 3.4 und dem dritten Armglied 3.3 durch das zweite Rotationsgelenk G3.4 möglich. Der Instrumentenhalter 3.4, den man auch als viertes Armglied bezeichnen könnte, ist also über zwei Gelenke, das Schubgelenk G3.3 und das zweite Rotationsgelenk G3.4, mit dem dritten Armglied verbunden. Diese Konstruktion erlaubt eine kompakte Bauform, die zudem mit wenigen und robust konstruierbaren Gelenken auskommt. Dies erhöht die Stabilität des gesamten Operationssystems. Da der Pivotpunkt PP nicht direkt in der Instrumententrägervorrichtung liegt, sind zur Realisierung der geforderten Positionierungsmöglichkeiten weniger Stellbewegungen notwendig, der benötigte Platz ist geringer. Zudem ist das Kollisionspotential mit den anderen Manipulatoren des robotischen Operationssystems auf ein Minimum reduziert. Der Pivotpunkt PP liegt auf der Verlängerung der Drehachse des Torsionsgelenks G3.1 deutlich außerhalb des Instrumententrägers, typischerweise sollte der Mindestabstand der Instrumententrägervorrichtung 3 zum Pivotpunkt PP entlang der Drehachse des Torsionsgelenks G3.1 etwa 10 cm betragen.

Zur Führung des Instruments im Pivotpunkt PP sind in der Regel keine weiteren Hilfsmittel notwendig, wenn der Pivotpunkt PP beispielsweise durch eine Körperöffnung in der Gewebedecke wie der Bauchdecke definiert werden kann. In der von der Bauchdecke bzw. Gewebedecke definierten Ebene erfolgt bei der Operation keine seitliche Bewegung durch die Instrumentenstange. In Situationen, wo ein solcher Pivotpunkt PP nicht durch eine entsprechende Gewebedecke definiert werden kann, ist es vorteilhaft, wenn am zweiten Armglied 3.2 im Bereich des ersten Rotationsgelenks G3.2 entlang der Verlängerung der Drehachse des Torsionsgelenks G3.1 - gekennzeichnet durch die gestrichelte Linie, welche entlang der Längsachse des ersten Armglieds 3.1 und des zweiten Armglieds 3.2 verläuft und auf welcher der Pivotpunkt PP liegt - eine Trokarhalterung 6 angeordnet ist. Diese ist zur schwenkbaren Lagerung eines Trokars um eine zur Rotationsachse des ersten Rotationsgelenks G3.2 parallele Schwenkachse P ausgebildet. Diese Trokarhalterung 6 kann beispielsweise auf einen entsprechenden Adapter, der am Ende des zweiten Armglieds 3.2 ausgebildet ist, aufgesteckt werden und mit einer Rastverbindung in der aufgesteckten Position fixiert werden. Auch andere Verbindungen sind denkbar, beispielsweise eine Stecker-Buchse-Verbindung oder eine Schraubverbindung. Die Verbindung ist kraft- und / oder formschlüssig. Die Instrumentenstange 5 wird hier durch eine entsprechende Lagerung auf der Schwenkachse P geführt, diese Lagerung kann in einem form- jedoch nicht kraftschlüssigen Halten der Instrumentenstange 5 bestehen, da diese entlang ihrer Längsachse verschiebbar gehalten werden muss. Beispielsweise kann ein freies Gelenk als Schwenklagerung GP vorgesehen sein, welches eine Durchbohrung aufweist, durch die die Instrumentenstange 5 geführt werden kann.

Die kinematischen Verhältnisse sind in Fig. 2 genauer dargestellt. Angetriebene Gelenke sind durch die entsprechenden Doppelpfeile gekennzeichnet. Nicht angetrieben sind das zweite Rotationsgelenk G3.4, die Schwenklagerung GP und die Führung der Instrumentenstange 5 durch den Pivotpunkt PP hindurch, diese Bewegung wird durch das Schubgelenk G3.3 in Verbindung mit den zweiten Rotationsgelenk G3.4 hervorgerufen.

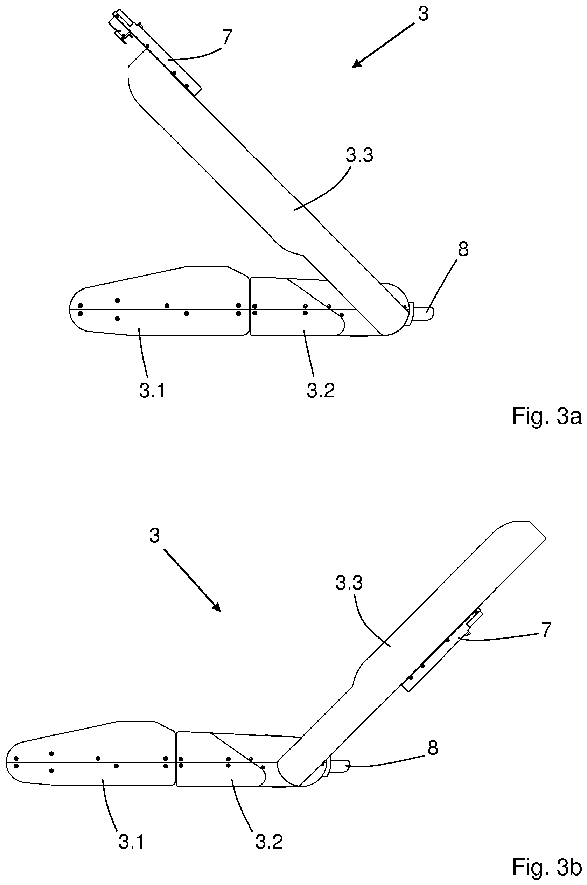

Fig. 3a und 3b zeigen eine Instrumententrägervorrichtung 3, allerdings ohne Instrumentenhalter 3.4, in zwei verschiedenen Stellungen des dritten Armglieds 3.3. In Fig. 3a ist eine negative Auslenkung und in Fig. 3b eine positive Auslenkung des ersten Rotationsgelenks G3.2 gezeigt. Ein Schwenken des dritten Armglieds 3.3 geht einher mit einer Verstellung des Schubgelenks G3.3, dieses Schubgelenk G3.3 führt einen Schlitten 7, auf welchem der Instrumentenhalter 3.4 angeordnet werden kann bzw. mit welchem dieser verbunden werden kann. Das zweite Rotationsgelenk G3.2 ist nicht am Schlitten 7 ausgebildet, sondern am Instrumentenhalter 3.4, es kann aber in einer konstruktiven Abwandlung auch ohne weiteres am Schlitten 7 ausgebildet werden. In der Fortsetzung des zweiten Armgliedes 3.2 auf der dem ersten Armglied 3.1 abgewandten Seite ist außerdem ein Adapter 8 zur Verbindung mit der Trokarhalterung 6 dargestellt.

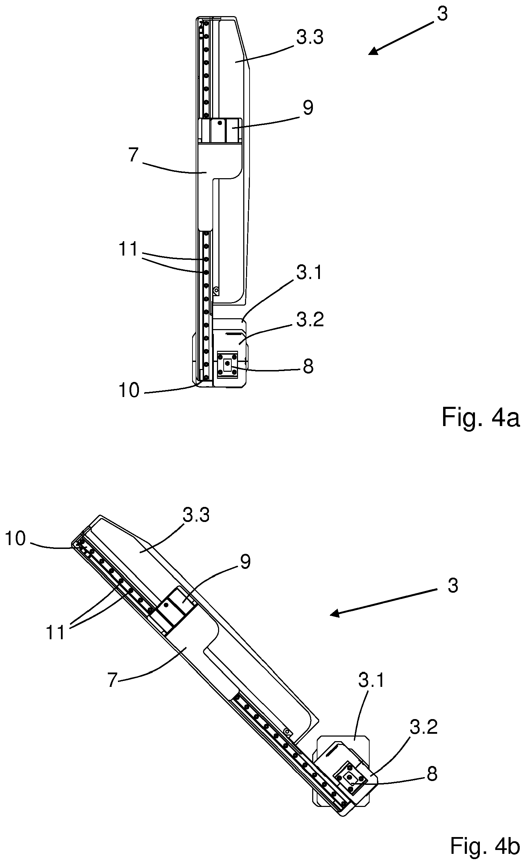

Fig. 4a und 4b zeigen die Instrumententrägervorrichtung 3 in einer anderen Ansicht, bei welcher das Armglied 3.3 im Detail dargestellt ist. In Fig. 4a ist das Torsionsgelenk G3.1 und damit das zweite Armglied 3.2 bezogen auf eine Torsion gegenüber dem ersten Armglied 3.1 in Ruhestellung - auch als Nulllage bezeichnet -, d.h. die Drehachsen des Schnittstellenrotationsgelenks GS und des ersten Rotationsgelenks G3.2 liegen parallel. In Fig. 4b ist das zweite Armglied 3.2 in einer anderen Position gegenüber der Position in Fig. 4a, somit auch das gegenüber dem zweiten Armglied 3.2 nicht bewegte dritte Armglied 3.3. Auch hier wurde auf eine Darstellung des Instrumentenhalters 3.4 verzichtet. Das Schubgelenk ist vorzugsweise als Linearführung 10 mit Spindelantrieb und/ oder Riemenantrieb ausgestaltet, diese Ausführung ist sehr robust. Die Position des Schlittens 7 entlang einer Schubgelenkachse - entsprechend der Längsachse des dritten Armglieds 3.3 und parallel zur Linearführung 10 - ist mittels Relativencoder bestimmbar, entlang der Schubgelenkachse sind dazu Referenzlagengeber 11 angeordnet. Durch den Antrieb wird der Schlitten 7 in der Linearführung 10 entlang der Längsausdehnung des dritten Armgliedes 3.3 bewegt. Auf dem Schlitten 7 befindet sich außerdem ein Instrumentenkoppler 9, hier kann der Instrumentenhalter 3.4 angekoppelt werden.

Eine Instrumententrägervorrichtung 3 mit angekoppeltem Instrumentenhalter 3.4 und darin aufgenommenen Instrument ist in Fig.5 dargestellt. Das dritte Armglied 3.3 bzw. das erste Rotationsgelenk G3.2 sind hier in Ruhestellung. Die Instrumentenstange 5 wird durch einen Trokar, genauer gesagt durch den Tubus 12 eines Trokars geführt. Dieser wird in die Gewebedecke eingeführt. Der Adapter 8 wird in diesem Falle nicht benötigt. Die Steuersignale für das Instrument werden über Antennen 13 übertragen, auf dem Schlitten 7 können dazu kleine Sender zur Signalübertragung per Funk angeordnet sein. Die Übertragung der Signale per Funk ist vorteilhaft, da ansonsten Leitungen verwendet werden müssten, deren mechanische Stabilität durch Schwenken um die Rotationsachse des zweiten Rotationsgelenks G3.4 beansprucht und leicht verschlissen werden können. Am Instrumentenhalter 3.4 ist außerdem ein Taster 14 angeordnet. Dieser kann beispielsweise als mechanischer Taster ausgestaltet sein, um den Instrumentenkopf 4 aus dem Instrumentenhalter 3.4 zu lösen, welcher hier durch einen Rastmechanismus 15 in Position gehalten wird, oder auch um die Motorbremse zu lösen und ein händisches Verfahren des Schlittens 7 zu ermöglichen, wenn beispielsweise das Instrument gewechselt oder entfernt werden soll. Die Instrumentenlängsachse I entspricht der Symmetrieachse der Instrumentenstange 5 des chirurgischen Instruments.

Auch der Instrumentenhalter 3.4 kann mittels eines Rastmechanismus mit dem dritten Armglied 3.3 verbunden werden, dies ermöglicht einen einfachen und schnellen An- und Abbau. Auch andere Verbindungen sind möglich, beispielsweise eine Schraubverbindung.

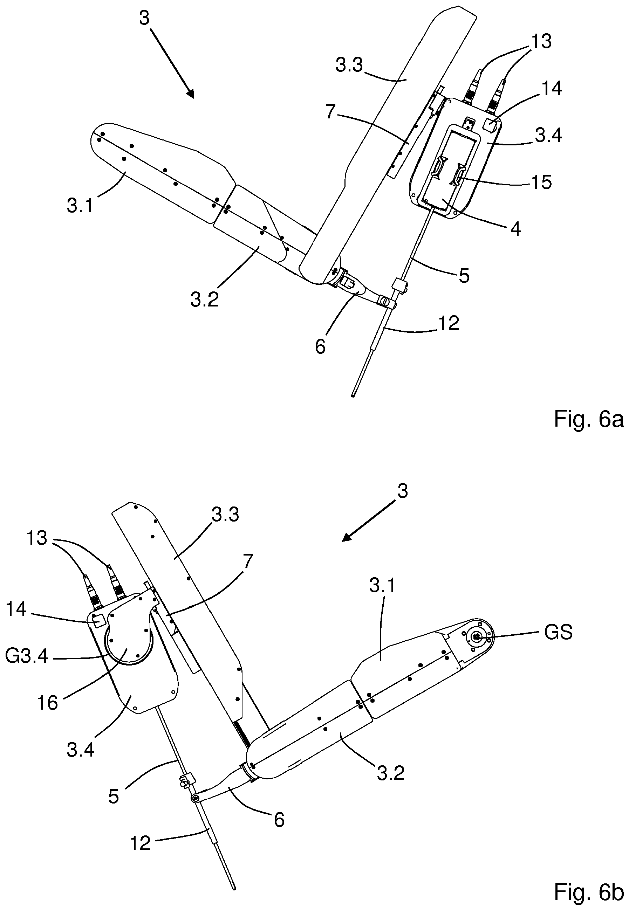

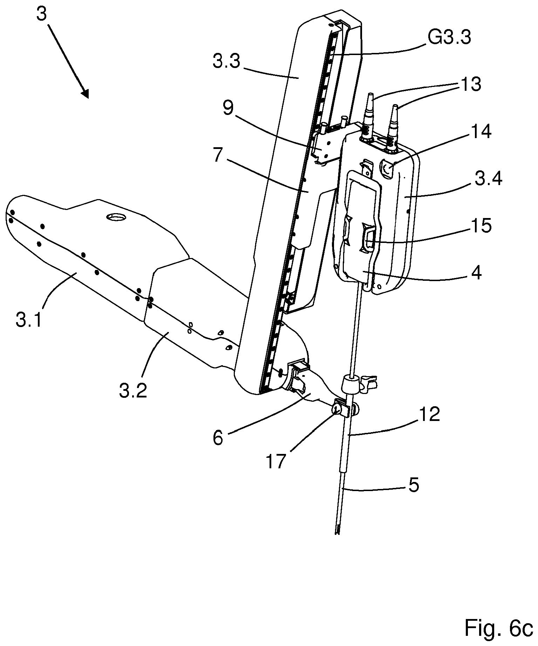

Während bei der in Fig. 5 gezeigt Ausführung keine Trokarhalterung 6 verwendet wird, ist dies bei der in Fig. 6a und 6b gezeigten Ausführung der Fall. Fig. 6a und 6b zeigen eine Instrumententrägervorrichtung 3 aus zwei entgegengesetzten Blickrichtungen, in Fig. 6b ist die Rückseite des Instrumentenhalters 3.4 dargestellt. Auch auf der Rückseite befindet sich ein weiterer Taster 14, der eine andere Funktion als der Taster 14 auf der Vorderseite haben kann. Er kann aber auch mit dem Taster 14 auf der Vorderseite gekoppelt sein, so dass sich beispielsweise nur durch Drücken auf beide Taster 14 gemeinsam der Rastmechanismus öffnet und den Instrumentenkopf freigibt, oder die Motorbremse gelöst wird. Der Instrumentenhalter 3.4 ist über das Gelenk G3.4 und eine Gelenkhalterung 16 mit dem Schlitten 7 verbunden. Fig. 6c zeigt die in Fig. 6a und 6b gezeigte Instrumententrägervorrichtung 3 in einer Perspektivansicht. Der Tubus 12 wird hier in einer an der Trokarhalterung 6 ausgebildeten Klemmvorrichtung 17 schwenkbar fixiert.

Fig. 7a, 7b zeigen schließlich eine Instrumententrägervorrichtung 3 mit einem anderen Instrumentenhalter 3.4, an welchem ein Endoskop 18 mit einer Endoskopstange 19 gekoppelt ist. Diese - auch für andere chirurgische Instrumente geeignete Bauform des Instrumentenhalters 3.4 benötigt weniger Platz, da sich das Gelenk aufgrund der gegenüber der in Fig. 6 gezeigten Ausgestaltung um 90° gedrehten Anordnung besser in den Instrumentenhalter 3.4 integrieren lässt. Auch das Endoskop 18 weist eine Instrumentenlängsachse I auf, welche der Symmetrieachse der Endoskopstange 19 entspricht.

Die vorangehend beschriebenen Vorrichtung ermöglicht es, ein robotisches Operationssystem, insbesondere einen Manipulator für ein robotisches Operationssystem kompakt und robust aufzubauen, was die Bedingung des Operationssystems durch den Operateur, den Chirurgen, erleichtert und die Belastung für den Patienten aufgrund der zwangsfreien Lagerung des Instruments bzw. Trokars im Pivotpunkt verringert.

### Bezugszeichenliste

- 1: Manipulator
- 2: Stellvorrichtung
- 2.1 - 2.4: Stellarmglieder
- 3: Instrumententrägervorrichtung
- 3.1 - 3.3: Armglied
- 3.4: Instrumentenhalter
- 4: Instrumentenkopf
- 5: Instrumentenstange
- 6: Trokarhalterung
- 7: Schlitten
- 8: Adapter
- 9: Instrumentenkoppler
- 10: Linearführung
- 11: Referenzlagergeber
- 12: Tubus
- 13: Antenne
- 14: Taster
- 15: Rastmechanismus
- 16: Gelenkhalterung
- 17: Klemmvorrichtung
- 18: Endoskop
- 19: Endoskopstange
- GP: Schwenklagerung
- GS: Schnittstellenrotationsgelenk
- G0: Haltergelenk
- G2.1 - G2.4: Stellvorrichtungsgelenk
- G3.1: Torsionsgelenk
- G3.2: erstes Rotationsgelenk
- G3.3: Schubgelenk
- G3.4: zweites Rotationsgelenk
- I: Instrumentenlängsachse
- PP: Pivotpunkt

## Patentansprüche

1. Instrumententrägervorrichtung (3) für einen Manipulator (1) eines robotischen Operationssystems, umfassend
- einen dreigliedrigen Arm als Endstück einer offenen kinematischen Kette mit einem ersten, zweiten und dritten Armglied (3.1, 3.2, 3.3),
- eine Schnittstelle zur Verbindung des ersten Armglieds (3.1) mit dem Manipulator (1) über ein Schnittstellenrotationsgelenk (GS),
- ein Torsionsgelenk (G3.1), welches das erste Armglied (3.1) mit dem zweiten Armglied (3.2) verbindet,
- ein erstes Rotationsgelenk (G3.2), welches das zweite Armglied (3.2) mit dem dritten Armglied (3.3) verbindet,
- ein Schubgelenk (G3.3), welches das dritte Armglied (3.3) mit einem Instrumentenhalter (3.4) zur Aufnahme eines chirurgischen Instruments (4, 5, 18, 19) mit einer Instrumentenlängsachse (I) verbindet, sowie
- eine Steuerung mit Antrieben für das Torsionsgelenk (G3.1), das erste Rotationsgelenk (G3.2) und das Schubgelenk (G3.3) zur Bewegung der Armglieder (3.1, 3.2, 3.3)und des Instrumentenhalters (3.4) relativ zueinander, **dadurch gekennzeichnet, dass**
- der Instrumentenhalter (3.4) mit dem dritten Armglied (3.3) über ein zweites, passives Rotationsgelenk (G3.4) verbunden und um dessen Rotationsachse frei rotierbar ist, wobei die Rotationsachsen des ersten Rotationsgelenks (G3.2) und des zweiten Rotationsgelenks (G3.4) zueinander parallel liegen, wodurch die Instrumentenlängsachse (I) zwangsfrei durch einen Pivotpunkt (PP) führbar ist, welcher auf der Drehachse des Torsionsgelenks (G3.1) in einer Verlängerung aus dem zweiten Armglied (3.2) heraus liegt.

2. Instrumententrägervorrichtung (3) nach Anspruch 1, **dadurch gekennzeichnet, dass** am zweiten Armglied (3.2) im Bereich des ersten Rotationsgelenks (G3.2) entlang der Verlängerung der Drehachse des Torsionsgelenks (G3.1) eine Trokarhalterung (6) angeordnet ist, welche zur schwenkbaren Lagerung eines Trokars um eine zur Rotationsachse des ersten Rotationsgelenks parallele Schwenkachse ausgebildet ist.

3. Instrumententrägervorrichtung (3) nach Anspruch 1 oder 2, **dadurch gekennzeichnet, dass** das Schubgelenk (G3.3) als Linearführung (10) mit Spindelantrieb ausgestaltet ist.

4. Instrumententrägervorrichtung (3) nach einem der Ansprüche 1 bis 3, **dadurch gekennzeichnet, dass** die Position des Schubgelenks (G3.3) entlang einer Schubgelenkachse mittels Relativencoder und entlang der Schubgelenkachse angeordneten Referenzlagengebern (11) bestimmbar ist.

5. Instrumententrägervorrichtung (3) nach einem der Ansprüche 1 bis 4, **dadurch gekennzeichnet, dass** der Instrumentenhalter (3.4) mittels eines Rastmechanismus mit dem dritten Armglied verbindbar ist.

6. Instrumententrägervorrichtung (3) nach einem der Ansprüche 1 bis 5, **dadurch gekennzeichnet, dass** an den Instrumentenhalter (3.4) ein Endoskop (18) mit einer Endoskopstange (19) gekoppelt ist.

## Claims

1. An instrument support device (3) for a manipulator (1) of a robotic surgical system, comprising
- a three-link arm as the end piece of an open kinematic chain with a first, second and third arm link (3.1, 3.2, 3.3),
- an interface for connecting the first arm link (3.1) to the manipulator (1) via an interface rotation joint (GS),
- a torsion joint (G3.1), which connects the first arm link (3.1) to the second arm link (3.2),
- a first rotation joint (G3.2), which connects the second arm link (3.2) to the third arm link (3.3),
- a thrust joint (G3.3) which connects the third arm link (3.3) to an instrument holder (3.4) for holding a surgical instrument (4, 5, 18, 19) with a longitudinal instrument axis (l), and
- a control system with drives for the torsion joint (G3.1), the first rotation joint (G3.2) and the thrust joint (G3.3) for moving the arm links (3.1, 3.2, 3.3) and the instrument holder (3.4) relative to one another, **characterised in that**
- the instrument holder (3.4) is connected to the third arm link (3.3) via a second, passive rotation joint (G3.4) and is freely rotatable about the axis of rotation of the latter, the axes of rotation of the first rotation joint (G3.2) and of the second rotation joint (G3. 4) being parallel to each other, which allows the longitudinal instrument axis (l) to be guided without constraint through a pivot point (PP), which lies on the axis of rotation of the torsion joint (G3.1) in an extension out of the second arm link (3.2).

2. The instrument support device (3) according to claim 1, **characterised in that** a trocar holder (6) is arranged on the second arm link (3.2) in the region of the first rotation joint (G3.2) along the extension of the axis of rotation of the torsion joint (G3.1), which trocar holder (6) is designed for the pivotable mounting of a trocar about a pivot axis parallel to the axis of rotation of the first rotation joint.

3. The instrument support device (3) according to claim 1 or 2, **characterised in that** the thrust joint (G3.3) is designed as a linear guide (10) with a spindle drive.

4. The instrument support device (3) according to any one of claims 1 to 3, **characterised in that** the position of the thrust joint (G3.3) along a thrust joint axis can be determined by means of a relative encoder and reference position encoders (11) arranged along the thrust joint axis.

5. The instrument support device (3) according to any one of claims 1 to 4, **characterised in that** the instrument holder (3.4) can be connected to the third arm link by means of a latching mechanism.

6. The instrument support device (3) according to any one of claims 1 to 5, **characterised in that** an endoscope (18) with an endoscope rod (19) is coupled to the instrument holder (3.4).

## Revendications

1. Ensemble porte-instrument (3) pour un manipulateur (1) d'un système robotisé pour interventions chirurgicales, comprenant
- un bras à trois membres comme élément d'extrémité d'une chaîne cinématique ouverte, comprenant un premier, un deuxième et un troisième membres de bras (3.1, 3.2, 3.3),
- une interface pour relier le premier membre de bras (3.1) au manipulateur (1) via une articulation rotative d'interface (GS),
- une articulation de torsion (G3.1) qui relie le premier membre de bras (3.1) au deuxième membre de bras (3.2),
- une première articulation de rotation (G3.2) qui relie le deuxième membre de bras (3.2) au troisième membre de bras (3.3),
- une articulation de poussée (G3.3) qui relie le troisième membre de bras (3.3) à un support d'instrument (3.4) destiné à recevoir un instrument chirurgical (4, 5, 18, 19) avec un axe longitudinal d'instrument (l), ainsi que
- une commande avec des entraînements pour l'articulation de torsion (G3.1), la première articulation de rotation (G3.2) et l'articulation de poussée (G3.3) pour le mouvement des membres de bras (3.1, 3.2, 3.3) et du support d'instrument (3.4) les uns par rapport aux autres, **caractérisé en ce que**
- le support d'instrument (3.4) est relié au troisième membre de bras (3.3) par une deuxième articulation de rotation passive (G3.4) et peut tourner librement autour de l'axe de rotation de cette dernière, les axes de rotation de la première articulation de rotation (G3.2) et de la deuxième articulation de rotation (G3.4) étant parallèles l'un à l'autre, ce qui permet de guider l'axe longitudinal d'instrument (l) sans contrainte à travers un point de pivotement (PP) qui se trouve sur l'axe de rotation de l'articulation de torsion (G3.1) dans un prolongement hors du deuxième membre de bras (3.2).

2. Ensemble porte-instrument (3) selon la revendication 1, **caractérisé en ce que** qu'un support de trocart (6) est disposé sur le deuxième membre de bras (3.2) dans la zone de la première articulation de rotation (G3.2) le long du prolongement de l'axe de rotation de l'articulation de torsion (G3.1), lequel est conçu pour le montage pivotant d'un trocart autour d'un axe de pivotement parallèle à l'axe de rotation de la première articulation de rotation.

3. Ensemble porte-instrument (3) selon la revendication 1 ou 2, **caractérisé en ce que** l'articulation de poussée (G3.3) est conçue comme un guidage linéaire (10) avec un entraînement par broche.

4. Ensemble porte-instrument (3) selon l'une quelconque des revendications 1 à 3, **caractérisé en ce que** la position de l'articulation de poussée (G3.3) le long d'un axe d'articulation de poussée peut être déterminée au moyen d'un codeur relatif et de codeurs de position de référence (11) disposés le long de l'axe d'articulation de poussée.

5. Ensemble porte-instrument (3) selon l'une quelconque des revendications 1 à 4, **caractérisé en ce que** le support d'instrument (3.4) peut être relié au troisième membre de bras au moyen d'un mécanisme d'encliquetage.

6. Ensemble porte-instrument (3) selon l'une quelconque des revendications 1 à 5, **caractérisé en ce qu'**un endoscope (18) avec une tige d'endoscope (19) est couplé au support d'instrument (3.4).
